# EUROPEAN PATENT APPLICATION

(11) **EP 1 995 227 A1**
(43) Date of publication of application: **26.11.2008**
(21) Application number: 08251216.1
(22) Date of filing: 31.03.2008
(51) Int. Cl.: C07C 13/00, C07C 13/64, C07C 211/19, C07D 209/96, A61P 31/16

(54) **Spiro and other derivatives of diamondoids posessing therapeutic activity in the treatment of viral disorders**

(30) Priority: 24.05.2007 US 931967 P
(71) Applicant: Chevron U.S.A. Inc., San Ramon, CA 94583-2324 (US)
(72) Inventor: Kong, Deyuan, Richmond, California 94806 (US); Lam, Frederick W, Piedmont, California 94611 (US); Sciamanna, Steven F, Orinda, California 94863 (US); Shelton, Earl, Menlo Park, California (US); Liu, Shenggao, Hercules, California 94547 (US); Carlton, Robert M., Petaluma, California 94952 (US)
(74) Representative: Nash, David Allan

(57) **Abstract**

This invention relates to diamondoid derivatives which exhibit therapeutic activity. Specifically, the diamondoid derivatives herein exhibit therapeutic effects in the treatment of viral disorders. Also provided are methods of treatment, prevention and inhibition of viral disorders in a subject in need.

## Description

### BACKGROUND OF THE INVENTION

### Field of the Invention

This invention relates to spiro and other derivatives of diamondoids which exhibit therapeutic activity. Specifically, the spiro and other diamondoid derivatives herein exhibit therapeutic effects in the treatment of viral disorders. Also provided are methods of treatment, prevention and inhibition of viral disorders in a subject in need.

### State of the Art

Diamondoids are cage-shaped hydrocarbon molecules possessing rigid structures, resembling tiny fragments of a diamond crystal lattice. *See* Fort, Jr., et al., Adamantane: Consequences of the Diamondoid Structure, Chem. Rev., 64:277-300 (1964). Adamantane is the smallest member of the diamondoid series and consists of one diamond crystal subunit. Diamantane contains two diamond subunits, triamantane contain three, and so on.

Adamantane, which is currently commercially available, has been studied extensively with regard to thermodynamic stability and functionalization, as well as to properties of adamantane containing materials. It has been found that derivatives containing adamantane have certain pharmaceutical uses, including anti-viral properties and uses as blocking agents and protecting groups in biochemical syntheses. For example, *alpha*-methyl-1-adamantanemethylamine hydrochloride (Flumadine® (remantidine) Forest Pharmaceuticals, Inc.) and 1-aminoadamantane hydrochloride (Symmetrel® (amantadine) Endo Laboratories, Inc.) may be used to treat influenza. Adamantanes are also useful in the treatment of Parkinson diseases.

However, though research has addressed the application of adamantane derivatives, studies on derivatives of the other two lower diamondoids (diamantane or triamantane) are very limited. U.S. Patent No. 5,576,355 discloses the preparation of adamantane and diamantane alcohol, ketone, ketone derivatives, adamantyl amino acid, quaternary salt or combinations thereof which have antiviral properties. U.S. Patent No. 4,600,782 describes the preparation of substituted spiro[oxazolidine-5,2'-adamantane] compounds useful as antiinflammatory agent. U.S. Patent No. 3,657,273 discloses the preparation of antibiotic adamantane-1,3-dicarboxamides having antibacterial, antifungal, antialgal, antiprotozoal, and antiinflammatory properties, as well as having analgesic and antihypertensive properties.

New agents, compositions and methods for using these agents and compositions that inhibit and treat viral disorders are needed, which can be used alone or in combination with other agents.

### SUMMARY OF THE INVENTION

The present invention provides diamondoid derivatives which exhibit pharmaceutical activity in the treatment, inhibition, and prevention of viral disorders. In particular, the present invention relates to derivatives of diamantane and triamantane, which may be used in the treatment, inhibition, and prevention of viral disorders. In its composition aspects, diamantane derivatives within the scope of the present invention include compounds of Formula I and II and triamantane derivatives within the scope of the present invention include compounds of Formula III and IV.

In one of its composition aspects, this invention is directed to a compound of Formula I: 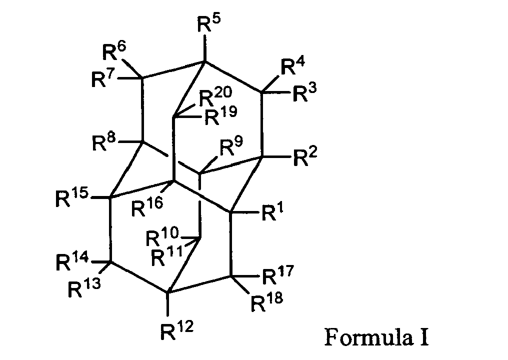 wherein:
R¹, R², R⁵, R⁸, R⁹, R¹², R¹⁵, and R¹⁶ are independently selected from the group consisting of hydrogen, hydroxy, lower alkyl, substituted lower alkyl, lower alkenyl, substituted lower alkenyl, alkoxy, amino, nitroso, nitro, halo, cycloalkyl, carboxy, acyloxy, acyl, aminoacyl, and aminocarbonyloxy;
R³, R⁴, R⁶, R⁷, R¹⁰, R¹¹, R¹³, R¹⁴, R¹⁷, R¹⁸, R¹⁹ and R²⁰ are hydrogen or R³ and R⁴, or R⁶ and R⁷, or R¹⁰ and R¹¹, or R¹³and R¹⁴, or R¹⁷ and R¹⁸, or R¹⁹ and R²⁰, together with the carbon to which they are bonded form a 3- to 7-membered ring structure having 0, 1, 2 or 3 heteroatoms ofN, O, P or S, the ring structure optionally substituted with one or more substituents independently selected from the group consisting of hydroxy, lower alkyl, substituted lower alkyl, lower alkenyl, substituted lower alkenyl, alkoxy, amino, nitroso, nitro, halo, cycloalkyl, carboxy, oxo, acyloxy, acyl, aminoacyl, and aminocarbonyloxy;
provided that at least one of R³ and R⁴, or R⁶ and R⁷, or R¹⁰ and R¹¹, or R¹³ and R¹⁴, or R¹⁷ and R¹⁸, or R¹⁹ and R²⁰, together with the carbon to which they are bonded form an optionally-substituted 3- to 7-membered ring structure;
and pharmaceutically acceptable salts thereof.

In another of its composition aspects, this invention is directed to a compound of Formula II: 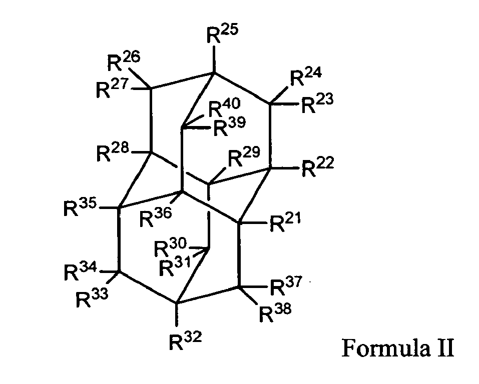 wherein:
R²¹, R²², R²⁵, R²⁸, R²⁹, R³², R³⁵, and R³⁶ are independently selected from the group consisting of hydrogen, hydroxy, lower alkyl, substituted lower alkyl, lower alkenyl, substituted lower alkenyl, alkoxy, amino, nitroso, nitro, halo, cycloalkyl, carboxy, acyloxy, acyl, aminoacyl, aminocarbonyloxy and a 3- to 7-membered ring structure having 0, 1, 2 or 3 heteroatoms ofN, O, P or S, the ring structure optionally substituted with one or more substituents independently selected from the group consisting of hydroxy, lower alkyl, substituted lower alkyl, lower alkenyl, substituted lower alkenyl, alkoxy, amino, nitroso, nitro, halo, cycloalkyl, carboxy, oxo, acyloxy, acyl, aminoacyl, and aminocarbonyloxy;
provided that at least one of R²¹, R²², R²⁵, R²⁸, R²⁹, R³², R³⁵, and R³⁶ is an optionally-substituted 3- to 7-membered ring structure;
R²³, R²⁴, R²⁶, R²⁷, R³⁰, R³¹, R³³, R³⁴, R³⁷, R³⁸, R³⁹, and R⁴⁰ are hydrogen;
and pharmaceutically acceptable salts thereof.

In one embodiment of the compounds of Formula I, only one of R³ and R⁴, or R⁶ and R⁷, or R¹⁰ and R¹¹, or R¹³ and R¹⁴, or R¹⁷ and R¹⁸, or R¹⁹ and R²⁰, together with the carbon to which they are bonded forms a ring structure. In another embodiment of the compounds of Formula I, two or more of R³ and R⁴ or R⁶ and R⁷ or R¹⁰ and R¹¹, or R¹³ and R¹⁴, or R¹⁷and R¹⁸, or R¹⁹ and R²⁰, together with the carbon to which they are bonded form a ring structure, and the ring structures contain at least one heteroatom.

In one embodiment of the compounds of Formula II, only one of R²¹, R²², R²¹, R²⁸, R²⁹, R³², R³⁵, and R³⁶ is a ring structure. In another embodiment of the compounds of Formula II, two or more of R²¹, R²², R²⁵, R²⁸, R²⁹, R³², R³⁵, and R³⁶ have a ring structure, and the ring structures contain at least one heteroatom.

In one preferred embodiment of the compounds of Formula I and the compounds of Fornula II, the ring structure has 1 or 2 heteroatoms. In another preferred embodiment of the compounds of Formula I and the compounds of Formula II, the ring structure has 1 heteroatom. In yet another preferred embodiment of the compounds of Formula I and the compounds of Formula II, the ring structure has 2 different heteroatoms. In still another preferred embodiment of the compounds of Formula I and the compounds of Formula II, the ring structure has 2 heteroatoms, both heteroatoms being the same.

In another embodiment of the compounds of Formula II, a tertiary carbon of the diamantane structure is joined to R²¹, R²², R²¹, R²⁸, R²⁹, R³², R³⁵, or R³⁶ by a carbon-carbon bond. In yet another embodiment of the compounds of Formula II, a tertiary carbon of the diamantane structure is joined to R²¹, R²², R²⁵ R²⁸ R²⁹, R³², R³⁵, or R³⁶ by a carbon-nitrogen bond.

In yet another embodiment of the compounds of Formula I and the compounds of Formula II, the ring structure is optionally substituted with lower alkyl, substituted lower alkyl, acyl, or cycloalkyl. In a preferred embodiment, the ring structure is optionally substituted with methyl, ethyl, propyl, acetyl, -NH₂, -NH(lower alkyl) or -N(lower alkyl)₂.

In a further embodiment of the compounds of Formula I and the compounds of Formula II, the ring structure is selected from the group consisting of azirane, azirine, azetane, azete, pyrrolidine, pyrrole, piperidine, pyridine, azepane and azepine.

In another embodiment of the compounds of Formula I and the compounds of Formula II, the ring structure is selected from the group consisting of oxirane, oxirene, oxetane, oxete, oxolane, furan, oxane, pyran, oxepane, oxepin and morpholine.

In yet another embodiment of the compounds of Formula I and the compounds of Formula II, the ring structure is selected from the group consisting of thiirane, thiirene, thietane, thiete, thiole, thiophene, thiane, thiine, thiepane and thiepin.

In still another embodiment of the compounds of Formula I and the compounds of Formula II, the ring structure is selected from the group consisting of cyclopropane, cyclopropene, cyclobutane, cyclobutene, cyclopentane, cyclopentene, cyclohexane, cyclohexene, benzene, cycloheptane and cycloheptene.

In yet another of its composition aspects, this invention is directed to compound of Formula III: 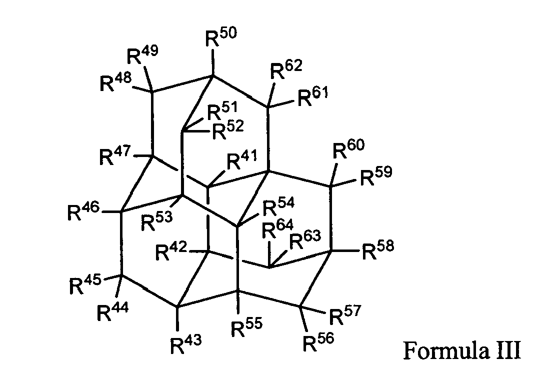 wherein:
R⁴¹, R⁴², R⁴³, R⁴⁶, R⁴⁷, R⁵⁰, R⁵³, R⁵⁴, R⁵⁵, and R⁵⁸ are independently selected from the group consisting of hydrogen, hydroxy, lower alkyl, substituted lower alkyl, lower alkenyl, substituted lower alkenyl, alkoxy, amino, nitroso, nitro, halo, cycloalkyl, carboxy, acyloxy, acyl, aminoacyl, and aminocarbonyloxy;
R⁴⁴, R⁴⁵, R⁴⁸, R⁴⁹, R⁵¹, R⁵², R⁵⁶, R⁵⁷, R⁵⁹, R⁶⁰, R⁶¹, R⁶², R⁶³, and R⁶⁴ are hydrogen or R⁴⁴ and R⁴⁵, or R⁴⁸ and R⁴⁹, or R⁵¹ and R⁵², or R⁵⁶ and R⁵⁷, or R⁵⁹ and R⁶⁰, or R⁶¹ and R⁶², or R⁶³ and R⁶⁴, together with the carbon to which they are bonded form a 3- to 7-membered ring structure having 0,1,2 or 3 heteroatoms of N, O, P or S, the ring structure optionally substituted with one or more substituents independently selected from the group consisting of hydroxy, lower alkyl, substituted lower alkyl, lower alkenyl, substituted lower alkenyl, alkoxy, amino, nitroso, nitro, halo, cycloalkyl, carboxy, oxo, acyloxy, acyl, aminoacyl, and aminocarbonyloxy;
provided that at least one of R⁴⁴ and R⁴⁵, or R⁴⁸ and R⁴⁹, or R⁵¹ and R⁵², or R⁵⁶ and R⁵⁷, or R⁵⁹ and R⁶⁰, or R⁶¹ and R⁶², or R⁶³ and R⁶⁴ together with the carbon to which they are bonded form an optionally-substituted 3- to 7-membered ring structure;
and pharmaceutically acceptable salts thereof.

In still another of its composition aspects, this invention if directed to a compound of Formula IV: 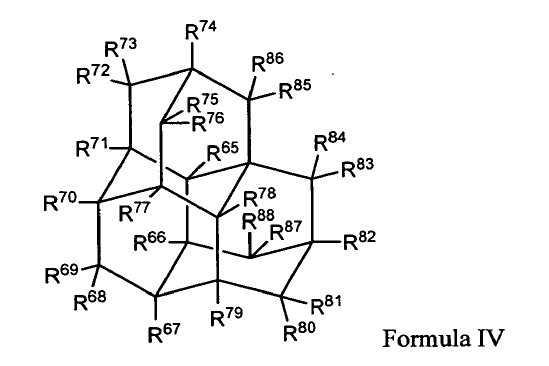 wherein:
R⁶⁵, R⁶⁶, R⁶⁷, R⁷⁰, R⁷¹, R⁷⁴, R⁷⁷, R⁷⁸, R⁷⁹, and R⁸² are independently selected from the group consisting of hydrogen, hydroxy, lower alkyl, substituted lower alkyl, lower alkenyl, substituted lower alkenyl, alkoxy, amino, nitroso, nitro, halo, cycloalkyl, carboxy, acyloxy, acyl, aminoacyl, aminocarbonyloxy and a 3- to 7-membered ring structure having 0, 1, 2 or 3 heteroatoms of N, O, P or S, the ring structure optionally substituted with one or more substituents independently selected from the group consisting of hydroxy, lower alkyl, substituted lower alkyl, lower alkenyl, substituted lower alkenyl, alkoxy, amino, nitroso, nitro, halo, cycloalkyl, carboxy, oxo, acyloxy, acyl, aminoacyl, and aminocarbonyloxy;
provided that at least one of R⁶⁵, R⁶⁶, R⁶⁷, R⁷⁰, R⁷¹, R⁷⁴, R⁷⁷, R⁷⁸, R⁷⁹, and R⁸² is an optionally-substituted 3- to 7-membered ring structure;
R⁶⁸, R⁶⁹, R⁷², R⁷³, R⁷⁵, R⁷⁶, R⁸⁰, R⁸¹, R⁸³, R⁸⁴, R⁸⁶, R⁸⁶, R⁸⁷, and R⁸⁸ are hydrogen;
and pharmaceutically acceptable salts thereof.

In one embodiment of the compounds of Formula III, only one of R⁴⁴ and R⁴⁵, or R⁴⁸ and R⁴⁹, or R⁵¹ and R⁵², or R⁵⁶ and R⁵⁷, or R⁵⁹ and R⁶⁰, or R⁶¹ and R⁶², or R⁶³ and R⁶⁴, together with the carbon to which they are bonded forms a ring structure. In another embodiment of the compounds of Formula III, two or more of R⁴⁴ and R⁴⁵, or R⁴⁸ and R⁴⁹, or R⁵¹ and R⁵², or R⁵⁶ and R⁵⁷, or R⁵⁹ and R⁶⁰, or R⁶¹ and R⁶², or R⁶³ and R⁶⁴, together with the carbon to which they are bonded form a ring structure, and the ring structures contain at least one heteroatom.

In one embodiment of the compounds of Formula IV, only one of R⁶⁵, R⁶⁶, R⁶⁷, R⁷⁰, R⁷¹, R⁷⁴, R⁷⁷, R⁷⁸, R⁷⁹, and R⁸² is a ring structure. In another embodiment of the compounds of Formula IV, two or more of R⁶⁵, R⁶⁶, R⁶⁷, R⁷⁰, R⁷¹, R⁷⁴, R⁷⁷, R⁷⁸, R⁷⁹, and R⁸² have a ring structure, and the ring structures contain at least one heteroatom.

In one preferred embodiment of the compounds of Formula III and the compounds of Fornula IV, the ring structure has 1 or 2 heteroatoms. In another preferred embodiment of the compounds of Formula III and the compounds of Formula IV, the ring structure has 1 heteroatom. In yet another preferred embodiment of the compounds of Formula III and the compounds of Formula IV, the ring structure has 2 different heteroatoms. In still another preferred embodiment of the compounds of Formula III and the compounds of Formula IV, the ring structure has 2 heteroatoms, both heteroatoms being the same.

In another embodiment of the compounds of Formula IV, a tertiary carbon of the diamantane structure is joined to R⁶⁵, R⁶⁶, R⁶⁷, R⁷⁰, R⁷¹, R⁷⁴, R⁷⁷, R⁷⁸, R⁷⁹, or R⁸² by a carbon-carbon bond. In yet another embodiment of the compounds of Formula IV, a tertiary carbon of the diamantane structure is joined to R⁶⁵, R⁶⁶, R⁶⁷, R⁷⁰, R⁷¹, R⁷⁴, R⁷⁷, R⁷⁸, R⁷⁹, or R⁸² by a carbon-nitrogen bond.

In yet another embodiment of the compounds of Formula III and the compounds of Formula IV, the ring structure is optionally substituted with lower alkyl, substituted lower alkyl, acyl, or cycloalkyl. In a preferred embodiment, the ring structure is optionally substituted with methyl, ethyl, propyl, acetyl, -NH₂, -NH(lower alkyl) or -N(lower alkyl)₂.

In a further embodiment of the compounds of Formula III and the compounds of Formula IV, the ring structure is selected from the group consisting of azirane, azirine, azetane, azete, pyrrolidine, pyrrole, piperidine, pyridine, azepane and azepine.

In another embodiment of the compounds of Formula III and the compounds of Formula IV, the ring structure is selected from the group consisting of oxirane, oxirene, oxetane, oxete, oxolane, furan, oxane, pyran, oxepane, oxepin and morpholine.

In yet another embodiment of the compounds of Formula III and the compounds of Formula IV, the ring structure is selected from the group consisting of thiirane, thiirene, thietane, thiete, thiole, thiophene, thiane, thiine, thiepane and thiepin.

In still another embodiment of the compounds of Formula III and the compounds of Formula IV, the ring structure is selected from the group consisting of cyclopropane, cyclopropene, cyclobutane, cyclobutene, cyclopentane, cyclopentene, cyclohexane, cyclohexene, benzene, cycloheptane and cycloheptene.

In one aspect, this invention provides for a method for treating a viral disorder in a subject in need thereof, comprising administering a therapeutically effective amount of a compound of Formula I, a compound of Formula II, a compound of Formula III, or a compound of Formula IV, as defined above.

In a preferred embodiment, the viral disorder is influenza. Preferably, the viral disorder may include influenza A and influenza B.

In another aspect, this invention provides pharmaceutical compositions comprising a pharmaceutically acceptable carrier and a therapeutically effective amount of the compounds defined herein.

In yet another aspect, the present invention provides processes for preparing compounds of Formula I, II, III and IV.

### DETAILED DESCRIPTION OF THE INVENTION

As described above, this invention relates to diamondoid derivatives which exhibit pharmaceutical activity, useful for the treatment, inhibition, and/or prevention of viral conditions. However, prior to describing this invention in further detail, the following terms will first be defined.

### Definitions

In accordance with this detailed description, the following abbreviations and definitions apply. It must be noted that as used herein, the singular forms "a", "an", and "the" include plural referents unless the context clearly dictates otherwise. Thus, for example, reference to "compounds" includes a plurality of such compounds and reference to "the dosage" includes reference to one or more dosages and equivalents thereof known to those skilled in the art, and so forth.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates, which may need to be independently confirmed.

Unless otherwise stated, the following terms used in the specification and claims have the meanings given below:
"Halo" means fluoro, chloro, bromo, or iodo.
"Nitro" means the group -NO₂.
"Nitroso" means the group -NO.
"Hydroxy" means the group -OH.
"Carboxy" means the group -COOH.
"Lower alkyl" refers to monovalent alkyl groups having from 1 to 6 carbon atoms including straight and branched chain alkyl groups. This term is exemplified by groups such as methyl, ethyl, iso-propyl, n-propyl, n-butyl, *iso*-butyl, *sec*-butyl, *t*-butyl, n-pentyl and the like.
"Substituted lower alkyl" means an alkyl group with one or more substituents, preferably one to three substituents, wherein the substitutents are selected from the group consisting of amino, nitroso, nitro, halo, hydroxy, carboxy, acyloxy, acyl, aminoacyl, and aminocarbonyloxy.
"Lower alkenyl" means a linear unsaturated monovalent hydrocarbon radical of two to six carbon atoms or a branched monovalent hydrocarbon radical of three to eight carbon atoms containing at least one double bond, (-C=C-). Examples of alkenyl groups include, but are not limited to, allyl, vinyl, 2-butenyl, and the like.
"Substituted lower alkenyl" means an alkenyl group with one or more substituents, preferably one to three substituents, wherein the substitutents are selected from the group consisting of amino, nitroso, nitro, halo, hydroxy, carboxy, acyloxy, acyl, aminoacyl, and aminocarbonyloxy.
The term "cycloalkyl" refers to cyclic alkyl groups of from 3 to 6 carbon atoms having a single cyclic ring including, by way of example, cyclopropyl, cyclobutyl, cyclopentyl and cyclohexyl.
"Alkoxy" refers to the group "lower alkyl-O-" which includes, by way of example, methoxy, ethoxy, n-propoxy, iso-propoxy, n-butoxy, tert-butoxy, sec-butoxy, n-pentoxy, 1,2-dimethylbutoxy, and the like.
"Amino" refers to the group NR^{a}R^{b}, wherein R^{a} and R^{b} are independently selected from hydrogen, lower alkyl, substituted lower alkyl, and cycloalkyl.
"Oxo" refers to the group -C(O)-, whether in a straight chain or a ring structure.
"Acyloxy" refers to the groups H-C(O)O-, lower alkyl-C(O)O-, substituted lower alkyl-C(O)O-, lower alkenyl-C(O)O-, substituted lower alkenyl-C(O)O- and cycloalkyl-C(O)O-, wherein lower alkyl, substituted lower alkyl, lower alkenyl, substituted lower alkenyl, and cycloalkyl are as defined herein.
"Acyl" refers to the groups H-C(O)-, lower alkyl-C(O)-, substituted lower alkyl-C(O)-, lower alkenyl-C(O)-, substituted lower alkenyl-C(O)-, cycloalkyl-C(O)-, wherein lower alkyl, substituted lower alkyl, lower alkenyl, substituted lower alkenyl, and cycloalkyl are as defined herein.
"Aminoacyl" refers to the groups -NRC(O)lower alkyl, -NRC(O)substituted lower alkyl, -NRC(O)cycloalkyl, -NRC(O)lower alkenyl, and -NRC(O)substituted lower alkenyl, wherein R is hydrogen or lower alkyl and wherein lower alkyl, substituted lower alkyl, lower alkenyl, substituted lower alkenyl, and cycloalkyl are as defined herein.
"Aminocarbonyloxy" refers to the groups -NRC(O)O-lower alkyl, -NRC(O)O-substituted lower alkyl, -NRC(O)O-lower alkenyl, -NRC(O)O-substituted lower alkenyl, - NRC(O)O-cycloalkyl, wherein R is hydrogen or lower alkyl and wherein lower alkyl, substituted lower alkyl, lower alkenyl, substituted lower alkenyl, and cycloalkyl are as defined herein.
"Pharmaceutically acceptable carrier" means a carrier that is useful in preparing a pharmaceutical composition that is generally safe, non-toxic and neither biologically nor otherwise undesirable, and includes a carrier that is acceptable for veterinary use as well as human pharmaceutical use. "A pharmaceutically acceptable carrier" as used in the specification and claims includes both one and more than one such carrier.
"Viral disorder" means any condition, disease and/or disorder related to infection by a virus.
"Treating" or "treatment" of a disease includes:
   (1) preventing the disease, i.e., causing the clinical symptoms of the disease not to develop in a mammal that may be exposed to or predisposed to the disease but does not yet experience or display symptoms of the disease,
   (2) inhibiting the disease, *i.e.,* arresting or reducing the development of the disease or its clinical symptoms, or
   (3) relieving the disease, *i.e.,* causing regression of the disease or its clinical symptoms.
A "therapeutically effective amount" means the amount of a compound that, when administered to a mammal for treating a disease, is sufficient to effect such treatment for the disease. The "therapeutically effective amount" will vary depending on the compound, the disease and its severity and the age, weight, etc., of the mammal to be treated.
"Pharmaceutically acceptable salt" refers to pharmaceutically acceptable salts of a compound which salts are derived from a variety of organic and inorganic counter ions well known in the art and include, by way of example only, sodium, potassium, calcium, magnesium, ammonium, tetraalkylammonium, and the like; and when the molecule contains a basic functionality, salts of organic or inorganic acids, such as hydrochloride, hydrobromide, tartrate, mesylate, acetate, maleate, oxalate and the like. Preferably, the pharmaceutically acceptable salts are of inorganic acid salts, such as hydrochloride.
"Optional" or "optionally" means that the subsequently described event or circumstance may, but need not, occur, and that the description includes instances where the event or circumstance occurs and instances in which it does not. For example, "aryl group optionally mono- or di- substituted with an alkyl group" means that the alkyl may but need not be present, and the description includes situations where the aryl group is mono- or disubstituted with an alkyl group and situations where the aryl group is not substituted with the alkyl group.
The term "mammal" refers to all mammals including humans, livestock, and companion animals.

The compounds of the present invention are generally named according to the IUPAC or CAS nomenclature system. Abbreviations which are well known to one of ordinary skill in the art may be used (e.g., "Ph" for phenyl, "Me" for methyl, "Et" for ethyl, "h" for hour or hours and "rt" for room temperature).

In naming the compounds of the present invention, the numbering scheme used for the diamantane ring system (C₁₄H₂₀) is as follows: 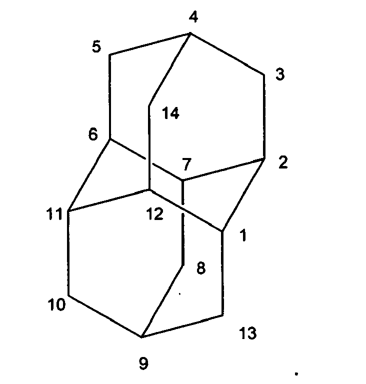 Positions 1, 2, 4, 6, 7, 9, 11, and 12 are bridgehead positions and the substituents at these positions are as defined for the compounds of Formula I and II. It is to be understood that in naming the compounds based upon the above positions, the compounds may be racemic mixtures of enantiomers (e.g., the enantiomers 1,6-dimethyl-2-amino diamantane and 1,6-dimethyl-12-amino diamantane and the enantiomers 1-methyl-7-amino diamantane and 1-methyl-11-amino diamantane).

In naming the compounds of the present invention, the numbering scheme used for the triamantane ring system (C₁₈H₂₄) is as follows: 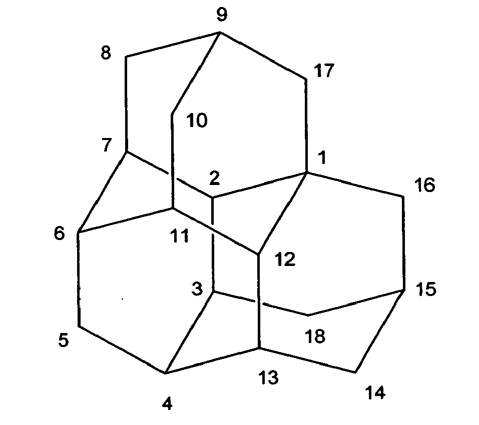 Positions 1, 2, 3, 4, 6, 7, 9, 11, 12, 13, and 15 are bridgehead positions and the substituents at these positions are as defined for the compounds of Formula III and IV.

### General Synthetic Schemes

Unsubstituted diamantane and triamantane may be synthesized by methods well known to those of skill in the art. For example, diamantane may be synthesized as described in Organic Syntheses, Vol 53, 30-34 (1973); Tetrahedron Letters, No. 44, 3877-3880 (1970); and Journal of the American Chemical Society, 87:4, 917-918 (1965). Triamantane may be synthesized as described in Journal of the American Chemical Society, 88:16, 3862-3863 (1966).

Furthermore, unsubstituted or alkylated diamantane and triamantane can be recovered from readily available feedstocks using methods and procedures well known to those of skill in the art. For example, unsubstituted or alkylated diamantane and triamantane can be isolated from suitable feedstock compositions by methods as described in U.S. Patent No. 5,414,189, herein incorporated by reference in its entirety. Furthermore, unsubstituted or alkylated diamantane and triamantane can be isolated from suitable feedstock compositions by methods as described for higher diamondoids in U.S. Patent No. 6,861,569, herein incorporated by reference in its entirety. It will be appreciated that where typical or preferred process conditions *(i.e.,* reaction temperatures, times, solvents, pressures, etc.) are given, other process conditions can also be used unless otherwise stated. Optimum reaction conditions may vary with feedstocks, but such conditions can be determined by one skilled in the art by routine optimization procedures. Suitable feedstocks are selected such that the feedstock comprises recoverable amounts of unsubstituted diamondoids selected from the group consisting of diamantane, triamanate, and mixtures thereof. Preferred feedstocks include, for example, natural gas condensates and refinery streams, including hydrocarbonaceous streams recoverable from cracking processes, distillations, coking, and the like. Preferred feedstocks include condensate fractions recovered from the Norphlet Formation in the Gulf of Mexico and from the LeDuc Formation in Canada.

Diamantane and triamantane, isolated as described above, may be derivatized to provide a compound of Formula I, II, III or IV according to the present invention by modification of synthetic pathways for preparing spiro-adamantane compounds and as described in further detail in the following examples.

Representative examples of diamantane and triamantane compounds derivatized with spiro- and/or 3- to 7-membered ring structures may be prepared from diamantane and triamantane, isolated as described above, by modifying synthetic pathways for preparing spiro- or heterocycle-substituted adamantane compounds, such as the procedures described in U.S. Patent No. 4,600,782; DE 1,965,481; Bioorg. Med. Chem., 14:3341-9, 2006; Bioorg. Med. Chem. Lett., 13:1699-1703, 2003; Bioorg. Med. Chem. Lett., 11:2137-2142, 2001; J. Med. Chem., 37:2896-2902, 1994; J. Med. Chem., 15:129-132, 1972; J. Med. Chem., 15:132-136, 1972; and J. Med. Chem., 39:3307, 1996. Derivatization of diamantane and triamantane compounds with hydroxy, lower alkyl, substituted lower alkyl, lower alkenyl, substituted lower alkenyl, alkoxy, amino, nitroso, nitro, halo, cycloalkyl, carboxy, acyloxy, acyl, aminoacyl, or aminocarbonyloxy groups may be performed as described in PCT application Serial No. xxxx [Attorney Docket No. 1005950-000947], filed April 17, 2007 and entitled" Diamondoid Derivatives Possessing Therapeutic Activity in the Treatment of Neurologic Disorders," herein incorporated by reference in its entirety.

The reagents used in preparing the compounds of Formula I, II, III, and IV are either available from commercial suppliers such as Toronto Research Chemicals (North York, ON Canada), Aldrich Chemical Co. (Milwaukee, Wisconsin, USA), Bachem (Torrance, California, USA), Emka-Chemie, or Sigma (St. Louis, Missouri, USA) or are prepared by methods known to those skilled in the art following procedures set forth in references such as Fieser and Fieser's Reagents for Organic Synthesis, Volumes 1-15 (John Wiley and Sons, 1991), Rodd's Chemistry of Carbon Compounds, Volumes 1-5 and Supplementals (Elsevier Science Publishers, 1989), Organic Reactions, Volumes 1-40 (John Wiley and Sons, 1991), March's Advanced Organic Chemistry, (John Wiley and Sons, 4th Edition), and Larock's Comprehensive Organic Transformations (VCH Publishers Inc., 1989). These schemes are merely illustrative of some methods by which the compounds of this invention can be synthesized, and various modifications to these schemes can be made and will be suggested to one skilled in the art having referred to this disclosure.

As it will be apparent to those skilled in the art, conventional protecting groups may be necessary to prevent certain functional groups from undergoing undesired reactions. Suitable protecting groups for various functional groups, as well as suitable conditions for protecting and deprotecting particular function groups are well known in the art. For example, numerous protecting groups are described in T.W. Greene and G.M. Wuts, Protecting Groups in Organic Synthesis, Second Edition, Wiley, New York, 1991, and references cited therein.

The starting materials and the intermediates of the reaction may be isolated and purified if desired using conventional techniques, including but not limited to filtration, distillation, crystallization, chromatography, and the like. Such materials may be characterized using conventional means, including physical constants and spectral data.

### Utility

The derivatives of diamantane and triamantane of the subject invention exhibit pharmaceutical activity, useful in the treatment, inhibition and/or prevention of viral disorders.

The diamantane and triamantane analogs of the present invention exhibit activity against viral disorders. Because diamantane and traimanatane are larger than adamantane, the diffusivity of diamantane, triamantane and their derivatives will be lower than that of adamantane and its corresponding derivatives.

In addition, substituting two amino groups onto the diamantane structure, as opposed to one amino group, improves the aqueous solubility, and decreases the lipid solubility, which will improve the bioavailability of the molecule. As diamantane and triamantane have rigid structures, they exhibit excellent bioavailability, as well as the ability to pass through the blood-brain barrier.

The compounds of the present invention may be used to treat, manage, and prevent viral disorders. The treatment of viral disorders has been addressed by methods which include inhibiting adsorption or penetration of virus into the cells, inhibiting intracellular processes which lead to the synthesis of viral components, or inhibition of release of newly synthesized virus from the infected cell. The inhibition of one or more of these steps depends on the chemistry or mode of action of the virus.

The term viral disorder embraces a collection of diseases and conditions, with each type consisting of numerous subsets. Viral disorders to be treated, inhibited, and/or prevented with the triamantane and diamantane derivatives set forth herein, include but are not limited to, those disorders caused by picornaviruses, rhinoviruses, enteroviruses, aphthoviruses, cardioviruses, hepatitis A virus, polioviruses, Coxsackieviruses, echoviruses, togaviruses, alphaviruses, rubiviruses, rubella virus, coronaviruses, rhabdoviruses, rabies virus, vesiculoviruses, paramyxoviruses, parainfluenza viruses, rubelaviruses, mumps virus, morbilliviruses, measels virus, pneumoviruses, respiratory syncytial viruses, orthomyxoviruses, influenza viruses including influenza A viruses, influenza B viruses and influenza C viruses, arboviruses, bunyaviruses, hantaviruses, nairoviruses, phleboviruses, arenaviruses, reoviruses, rotaviruses, retroviruses, lentiviruses, HTLV-1, HTLV-2, HIV-1, HIV-2, polyomaviruses, papillomaviruses, adenoviruses, parvoviruses, herpes simplex viruses, Epstein-Barr virus, varicella-zoster virus, poxviruses, variola virus, hepadnaviruses, hepatitis B virus, hepatitis C virus, cytomegaloviruses, flaviviruses, West Nile virus and dengue viruses. Preferrred viral disorders to be treated, inhibited, and/or prevented are those caused by influenza viruses, including influenza A viruses, influenza B viruses and influenza C viruses. More preferably, the viral disorders to be treated, inhibited, and/or prevented are those caused by influenza B viruses or influenza A viruses, including influenza A virus having serotype H1N1, H2N2, H3N2, H5N1, H7N7, H1N2, H9N2, H7N2, H7N3 or H10N7.

Amantadine and rimantadine are two adamantane derivatives which inhibit the ability of influenza A virus to replicate. These agents are believed to inhibit influenza A virus replication by blocking the ion channel of the virus membrane protein M2. Diamantane and triamantane derivatives set forth herein may also inhibit influenza A virus replication by interaction with the M2 protein.

### Pharmaceutical Formulations

In general, the compounds of the subject invention will be administered in a therapeutically effective amount by any of the accepted modes of administration for these compounds. The compounds can be administered by a variety of routes, including, but not limited to, oral, parenteral (e.g., subcutaneous, subdural, intravenous, intramuscular, intrathecal, intraperitoneal, intracerebral, intraarterial, or intralesional routes of administration), topical, intranasal, localized (e.g., surgical application or surgical suppository), rectal, and pulmonary (e.g., aerosols, inhalation, or powder). Accordingly, these compounds are effective as both injectable and oral compositions. Preferably, the compounds are administered by oral route. Also preferably, the compounds are administered by parenteral routes. The compounds can be administered continuously by infusion or by bolus injection. More preferably, the compounds are administered by intravenous routes. Such compositions are prepared in a manner well known in the pharmaceutical art.

The actual amount of the compound of the subject invention, i.e., the active ingredient, will depend on a number of factors, such as the severity of the disease, i.e., the condition or disease to be treated, the age and relative health of the subject, the potency of the compound used, the route and form of administration, and other factors.

Toxicity and therapeutic efficacy of such compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds that exhibit large therapeutic indices are preferred.

The data obtained from cell culture assays and animal studies can be used in formulating a range of dosage for use in humans and other animal patients. The dosage of such compounds lies preferably within a range of circulating concentrations that include the ED₅₀ with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range which includes the IC₅₀ *(i.e.,* the concentration of the test compound which achieves a half-maximal inhibition of symptoms) as determined in cell culture. Such information can be used to more accurately determine useful doses in humans. Levels in plasma may be measured, for example, by high performance liquid chromatography. The effective blood level of the compounds of the subject invention is preferably greater than or equal to 40 ng/ml.

The amount of the pharmaceutical composition administered to the patient will vary depending upon what is being administered, the purpose of the administration, such as prophylaxis or therapy, the state of the patient, the manner of administration, and the like. In therapeutic applications, compositions are administered to a patient already suffering from a disease in an amount sufficient to cure or at least partially arrest the symptoms of the disease and its complications. An amount adequate to accomplish this is defined as "therapeutically effective dose." Amounts effective for this use will depend on the disease condition being treated as well as by the judgment of the attending clinician depending upon factors such as the severity of the inflammation, the age, weight and general condition of the patient, and the like.

The compositions administered to a patient are in the form of pharmaceutical compositions described *supra.* These compositions may be sterilized by conventional sterilization techniques, or may be sterile filtered. The resulting aqueous solutions may be packaged for use as is, or lyophilized, the lyophilized preparation being combined with a sterile aqueous carrier prior to administration. The pH of the compound preparations typically will be between 3 and 11, more preferably from 5 to 9 and most preferably from 7 to 8. It will be understood that use of certain of the foregoing excipients, carriers, or stabilizers will result in the formation of pharmaceutical salts.

The active compound is effective over a wide dosage range and is generally administered in a pharmaceutically or therapeutically effective amount. The therapeutic dosage of the compounds of the present invention will vary according to, for example, the particular use for which the treatment is made, the manner of administration of the compound, the health and condition of the patient, and the judgment of the prescribing physician. For example, for oral administration, the dose will typically be in the range of about 5 mg to about 300 mg per day, preferably about 100 mg to about 200 mg per day. For intravenous administration, the dose will typically be in the range of about 0.5 mg to about 50 mg per kilogram body weight, preferably about 2 mg to about 20 mg per kilogram body weight. Effective doses can be extrapolated from dose-response curves derived *from in vitro* or animal model test systems. Typically, the clinician will administer the compound until a dosage is reached that achieves the desired effect.

When employed as pharmaceuticals, the compounds of the subject invention are usually administered in the form of pharmaceutical compositions. This invention also includes pharmaceutical compositions, which contain as the active ingredient, one or more of the compounds of the subject invention above, associated with one or more pharmaceutically acceptable carriers or excipients. The excipient employed is typically one suitable for administration to human subjects or other mammals. In making the compositions of this invention, the active ingredient is usually mixed with an excipient, diluted by an excipient or enclosed within a carrier which can be in the form of a capsule, sachet, paper or other container. When the excipient serves as a diluent, it can be a solid, semi-solid, or liquid material, which acts as a vehicle, carrier or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing, for example, up to 10% by weight of the active compound, soft and hard gelatin capsules, suppositories, sterile injectable solutions, and sterile packaged powders.

In preparing a formulation, it may be necessary to mill the active compound to provide the appropriate particle size prior to combining with the other ingredients. If the active compound is substantially insoluble, it ordinarily is milled to a particle size of less than 200 mesh. If the active compound is substantially water soluble, the particle size is normally adjusted by milling to provide a substantially uniform distribution in the formulation, *e.g.,* about 40 mesh.

Some examples of suitable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, sterile water, syrup, and methyl cellulose. The formulations can additionally include: lubricating agents such as talc, magnesium stearate, and mineral oil; wetting agents; emulsifying and suspending agents; preserving agents such as methyl- and propylhydroxy-benzoates; sweetening agents; and flavoring agents. The compositions of the invention can be formulated so as to provide quick, sustained or delayed release of the active ingredient after administration to the patient by employing procedures known in the art.

The quantity of active compound in the pharmaceutical composition and unit dosage form thereof may be varied or adjusted widely depending upon the particular application, the manner or introduction, the potency of the particular compound, and the desired concentration. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. The concentration of therapeutically active compound may vary from about 0.5 mg/ml to 500 g/ml.

Preferably, the compound can be formulated for parenteral administration in a suitable inert carrier, such as a sterile physiological saline solution. For example, the concentration of compound in the carrier solution is typically between about 1-100 mg/ml. The dose administered will be determined by route of administration. Preferred routes of administration include parenteral or intravenous administration. A therapeutically effective dose is a dose effective to produce a significant steroid tapering. Preferably, the amount is sufficient to produce a statistically significant amount of steroid tapering in a subject.

By way of example, for preparing solid compositions such as tablets, the principal active ingredient is mixed with a pharmaceutical excipient to form a solid preformulation composition containing a homogeneous mixture of a compound of the present invention. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation is then subdivided into unit dosage forms of the type described above containing from, for example, 0.1 to about 500 mg of the active ingredient of the present invention.

The tablets or pills of the present invention may be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer, which serves to resist disintegration in the stomach and permit the inner component to pass intact into the duodenum or to be delayed in release. A variety of materials can be used for such enteric layers or coatings, such materials including a number of polymeric acids and mixtures of polymeric acids with such materials as shellac, cetyl alcohol, and cellulose acetate.

The liquid forms in which the novel compositions of the present invention may be incorporated for administration orally or by injection include aqueous solutions, suitably flavored syrups, aqueous or oil suspensions, and flavored emulsions with edible oils such as corn oil, cottonseed oil, sesame oil, coconut oil, or peanut oil, as well as elixirs and similar pharmaceutical vehicles. Syrups are preferred.

Compositions for inhalation or insufflation include solutions and suspensions in pharmaceutically acceptable, aqueous or organic solvents, or mixtures thereof, and powders. The liquid or solid compositions may contain suitable pharmaceutically acceptable excipients as described *supra.* The compositions may be administered by the oral or nasal respiratory route for local or systemic effect. Compositions in preferably pharmaceutically acceptable solvents may be nebulized by use of inert gases. Nebulized solutions may be inhaled directly from the nebulizing device or the nebulizing device may be attached to a face mask tent, or intermittent positive pressure breathing machine. Solution, suspension, or powder compositions may be administered, preferably orally or nasally, from devices which deliver the formulation in an appropriate manner.

The compounds of this invention can be administered in a sustained release form. Suitable examples of sustained-release preparations include semipermeable matrices of solid hydrophobic polymers containing the protein, which matrices are in the form of shaped articles, e.g., films, or microcapsules. Examples of sustained-release matrices include polyesters, hydrogels (e.g., poly(2-hydroxyethyl-methacrylate) as described by Langer et al., J. Biomed. Mater. Res. 15: 167-277 (1981) and Langer, Chem. Tech. 12: 98-105 (1982) or poly(vinyl alcohol)), polylactides (U.S. Patent No. 3,773,919), copolymers of L-glutamic acid and gamma ethyl-L-glutamate (Sidman et al., Biopolymers 22: 547-5 56, 1983), nondegradable ethylene-vinyl acetate *(Langer et al., supra*), degradable lactic acid-glycolic acid copolymers such as the LUPRON DEPOT^{™} (*i.e.*, injectable microspheres composed of lactic acid-glycolic acid copolymer and leuprolide acetate), and poly-D-(-)-3-hydroxybutyric acid (EP 133,988).

The compounds of this invention can be administered in a sustained release form, for example a depot injection, implant preparation, or osmotic pump, which can be formulated in such a manner as to permit a sustained release of the active ingredient. Implants for sustained release formulations are well-known in the art. Implants may be formulated as, including but not limited to, microspheres, slabs, with biodegradable or non-biodegradable polymers. For example, polymers of lactic acid and/or glycolic acid form an erodible polymer that is well-tolerated by the host. The implant is placed in proximity to the site of protein deposits (e.g., the site of formation of amyloid deposits associated with neurodegenerative disorders), so that the local concentration of active agent is increased at that site relative to the rest of the body.

The following formulation examples illustrate pharmaceutical compositions of the present invention.

### Formulation Example 1

Hard gelatin capsules containing the following ingredients are prepared:

| | Quantity |
|---|---|
| Ingredient | (mg/capsule) |
| Active Ingredient | 30.0 |
| Starch | 305.0 |
| Magnesium stearate | 5.0 |

The above ingredients are mixed and filled into hard gelatin capsules in 340 mg quantities.

### Formulation Example 2

A tablet formula is prepared using the ingredients below:

| | Quantity |
|---|---|
| Ingredient | (mg/capsule) |
| Active Ingredient | 25.0 |
| Cellulose, microcrystalline | 200.0 |
| Colloidal silicon dioxide | 10.0 |
| Stearic acid | 5.0 |

The components are blended and compressed to form tablets, each weighing 240 mg.

### Formulation Example 3

A dry powder inhaler formulation is prepared containing the following components:

| Ingredient | Weight % |
|---|---|
| Active Ingredient | 5 |
| Lactose | 95 |

The active mixture is mixed with the lactose and the mixture is added to a dry powder inhaling appliance.

### Formulation Example 4

Tablets, each containing 30 mg of active ingredient, are prepared as follows:

| | Quantity |
|---|---|
| Ingredient | (mg/capsule) |
| Active Ingredient | 30.0 mg |
| Starch | 45.0 mg |
| Microcrystalline cellulose | 35.0 mg |
| Polyvinylpyrrolidone | 4.0 mg |
| (as 10% solution in water) | |
| Sodium carboxymethyl starch | 4.5 mg |
| Magnesium stearate | 0.5 mg |
| Talc | 1.0 mg |
| Total | 120 mg |

The active ingredient, starch and cellulose are passed through a No. 20 mesh U.S. sieve and mixed thoroughly. The solution of polyvinyl-pyrrolidone is mixed with the resultant powders, which are then passed through a 16 mesh U.S. sieve. The granules so produced are dried at 50° to 60°C and passed through a 16 mesh U.S. sieve. The sodium carboxymethyl starch, magnesium stearate, and talc, previously passed through a No. 30 mesh U.S. sieve, are then added to the granules, which after mixing, are compressed on a tablet machine to yield tablets each weighing 150 mg.

### Formulation Example 5

Capsules, each containing 40 mg of medicament are made as follows:

| | Quantity |
|---|---|
| Ingredient | (mg/capsule) |
| Active Ingredient | 40.0 mg |
| Starch | 109.0 mg |
| Magnesium stearate | 1.0 mg |
| Total | 150.0 mg |

The active ingredient, cellulose, starch, an magnesium stearate are blended, passed through a No. 20 mesh U.S. sieve, and filled into hard gelatin capsules in 150 mg quantities.

### Formulation Example 6

Suppositories, each containing 25 mg of active ingredient are made as follows:

| Ingredient | Amount |
|---|---|
| Active Ingredient | 25 mg |
| Saturated fatty acid glycerides | to 2,000 mg |

The active ingredient is passed through a No. 60 mesh U.S. sieve and suspended in the saturated fatty acid glycerides previously melted using the minimum heat necessary. The mixture is then poured into a suppository mold of nominal 2.0 g capacity and allowed to cool.

### Formulation Example 7

Suspensions, each containing 50 mg of medicament per 5.0 ml dose are made as follows:

| Ingredient | Amount |
|---|---|
| Active Ingredient | 50.0 mg |
| Xanthan gum | 4.0 mg |
| Sodium carboxymethyl cellulose (11%) | |
| Microcrystalline cellulose (89%) | 50.0 mg |
| Sucrose | 1.75 g |
| Sodium benzoate | 10.0 mg |
| Flavor and Color | q.v. |
| Purified water | to 5.0 ml |

The medicament, sucrose and xanthan gum are blended, passed through a No. 10 mesh U.S. sieve, and then mixed with a previously made solution of the microcrystalline cellulose and sodium carboxymethyl cellulose in water. The sodium benzoate, flavor, and color are diluted with some of the water and added with stirring. Sufficient water is then added to produce the required volume.

### Formulation Example 8

Hard gelatin tablets, each containing 15 mg of active ingredient are made as follows:

| | Quantity |
|---|---|
| Ingredient | (mg/capsule |
| Active Ingredient | 15.0 mg |
| Starch | 407.0 mg |
| Magnesium stearate | 3.0 mg |
| Total | 425.0 mg |

The active ingredient, cellulose, starch, and magnesium stearate are blended, passed through a No. 20 mesh U.S. sieve, and filled into hard gelatin capsules in 560 mg quantities.

### Formulation Example 9

An intravenous formulation may be prepared as follows:

| Ingredient | Quantity |
|---|---|
| Active Ingredient | 250.0 mg |
| Isotonic saline | 1000 ml |

Therapeutic compound compositions generally are placed into a container having a sterile access port, for example, an intravenous solution bag or vial having a stopper pierceable by a hypodermic injection needle or similar sharp instrument.

### Formulation Example 10

A topical formulation may be prepared as follows:

| Ingredient | Quantity |
|---|---|
| Active Ingredient | 1-10 g |
| Emulsifying Wax | 30 g |
| Liquid Paraffin | 20 g |
| White Soft Paraffin | to 100 g |

The white soft paraffin is heated until molten. The liquid paraffin and emulsifying wax are incorporated and stirred until dissolved. The active ingredient is added and stirring is continued until dispersed. The mixture is then cooled until solid.

### Formulation Example 11

An aerosol formulation may be prepared as follows:
A solution of the candidate compound in 0.5% sodium bicarbonate/saline (w/v) at a concentration of 30.0 mg/mL is prepared using the following procedure:
A. Preparation of 0.5% Sodium Bicarbonate / Saline Stock Solution: 100.0 mL

| **Ingredient** | **Gram / 100.0 mL** | **Final Concentration** |
|---|---|---|
| Sodium Bicarbonate | 0.5 g | 0.5% |
| Saline | q.s. ad 100.0 mL | q.s. ad 100% |

Procedure:
1. Add 0.5g sodium bicarbonate into a 100 mL volumetric flask.
2. Add approximately 90.0 mL saline and sonicate until dissolved.
3. Q.S. to 100.0 mL with saline and mix thoroughly.
B. Preparation of 30.0 mg/mL Candidate Compound: 10.0 mL

| **Ingredient** | **Gram /10.0 mL** | **Final Concentration** |
|---|---|---|
| Candidate Compound | 0.300 g | 30.0 mg/mL |
| 0.5% Sodium Bicarbonate / Saline Stock Solution | q.s. ad 10.0 mL | q.s ad 100% |

Procedure:
1. Add 0.300 g of the candidate compound into a 10.0 mL volumetric flask.
2. Add approximately 9.7 mL of 0.5% sodium bicarbonate / saline stock solution.
3. Sonicate until the candidate compound is completely dissolved.
4. Q.S. to 10.0 mL with 0.5% sodium bicarbonate / saline stock solution and mix thoroughly.

Another preferred formulation employed in the methods of the present invention employs transdermal delivery devices ("patches"). Such transdermal patches may be used to provide continuous or discontinuous infusion of the compounds of the present invention in controlled amounts. The construction and use of transdermal patches for the delivery of pharmaceutical agents is well known in the *art. See, e.g.,* U.S. Patent No. 5,023,252, issued June 11, 1991, herein incorporated by reference in its entirety for or all purposes. Such patches may be constructed for continuous, pulsatile, or on demand delivery of pharmaceutical agents.

Direct or indirect placement techniques may be used when it is desirable or necessary to introduce the pharmaceutical composition to the brain. Direct techniques usually involve placement of a drug delivery catheter into the host's ventricular system to bypass the blood-brain barrier. One such implantable delivery system used for the transport of biological factors to specific anatomical regions of the body is described in U.S. Patent No. 5,011,472, which is herein incorporated by reference in its entirety for all purposes.

Indirect techniques, which are generally preferred, usually involve formulating the compositions to provide for drug latentiation by the conversion of hydrophilic drugs into lipid-soluble drugs. Latentiation is generally achieved through blocking of the hydroxy, carbonyl, sulfate, and primary amine groups present on the drug to render the drug more lipid soluble and amenable to transportation across the blood-brain barrier. Alternatively, the delivery of hydrophilic drugs may be enhanced by intra-arterial infusion of hypertonic solutions which can transiently open the blood-brain barrier.

According to one aspect of the invention, the compound may be administered alone, as a combination of compounds, or in combination with anti-alpha-4-antibodies. The compounds of the present invention may also be administered in combination with an immunosuppressant, wherein the immunosuppressant is not a steroid, an anti-TNF composition, a 5-ASA composition, and combinations thereof, wherein the immunosuppressant, anti-TNF composition, and 5-ASA composition are typically used to treat the condition or disease for which the compound of the present invention is being administed. The immunosuppressant may be azathioprine, 6-mercaptopurine, methotrexate, or mycophenolate. The anti-TNF composition may be infliximab. The 5-ASA agent may be mesalazine or osalazine.

When administered in combination, the small compounds may be administered in the same formulation as these other compounds or compositions, or in a separate formulation. When administered in combinations, the steroid sparing agents may be administered prior to, following, or concurrently with the other compounds and compositions.

Pharmaceutical compositions of the invention are suitable for use in a variety of drug delivery systems. Suitable formulations for use in the present invention are found in REMINGTON'S PHARMACEUTICAL SCIENCES, Mace Publishing Company, Philadelphia, PA, 17th ed. (1985).

In order to enhance serum half-life, the compounds may be encapsulated, introduced into the lumen of liposomes, prepared as a colloid, or other conventional techniques may be employed which provide an extended serum half-life of the compounds. A variety of methods are available for preparing liposomes, as described in, e.g., Szoka et al., U.S. Patent Nos. 4,235,871, 4,501,728 and 4,837,028 each of which is incorporated herein by reference in its entirety for all purposes.

The following synthetic and biological examples are offered to illustrate this invention and are not to be construed in any way as limiting the scope of this invention.

### Example 1:

### General synthetic route for spiro-azetidine-diamantane derivatives

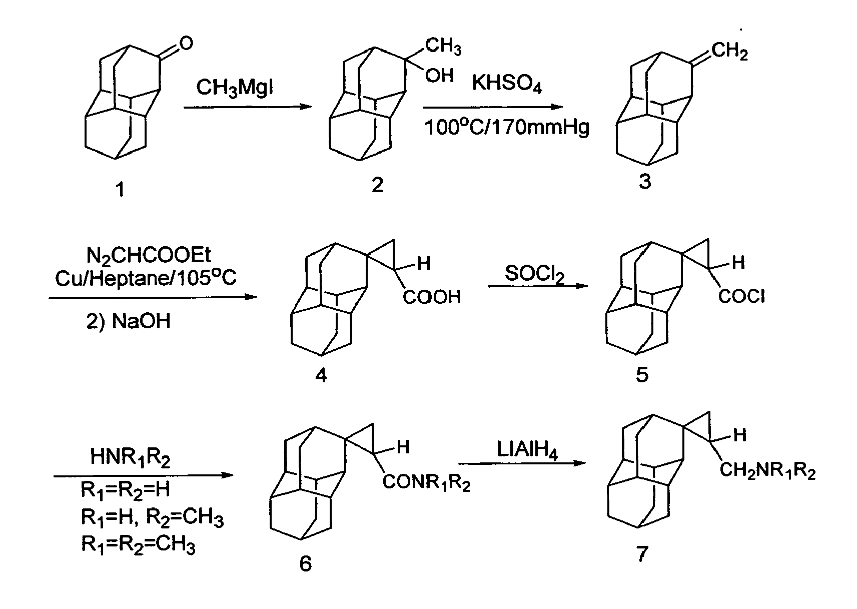

Compound **2** is synthesized by reaction of 3-diamantanone **(1)** with methyl magnesium iodide in dried dichloromethane. A mixture of 3-methyl-3-diamantanone **2** and anhydrous KHSO₄ is subjected to sublimation at 100°C (170 mm Hg), then the white solid of 3-methylene-diamantane **3** is collected.

A stirred mixture of **3** and freshly prepared and dried copper-bronze dust in heptane is heated to reflux at 105°C, and excess ethyl diazoacetate is then added dropwise over 30 min. The reaction is kept at 105°C for 1 hour then is run at room temperature for an additional 15 hours. The organic phase is separated and evaporated till dryness, and the residue is refluxed with a NaOH/water/ethanol mixture for 4 hours. After removal of ethanol, the water phase is acidified with 18% HCl to give the compound **4** in the carboxylic acid form.

A mixture of compound **4** and thionyl chloride is heated at 40°C for 0.5 hour then excess of thionyl chloride is removed. The resulting chloride **5** is used without purification. The chloride 5 in dried tetrahydrofurane is added dropwise to a 28% aqueous ammonia solution kept stirring for 10 min. Then water is added to precipitate the amide **6.** Compound **6** is washed with water to get rid of excess ammonia. In this step, an different alkyl amine (methylamine, dimethylamine) can be used to produce different amine products.

Compound **6** is reduced with LiA1H₄ in dry dimethyoxyethane under N₂. After work out, the final amine form of compound 7 is given.

### Example 2:

### General synthetic route for spiro-pyrrolidine-diamantane derivatives

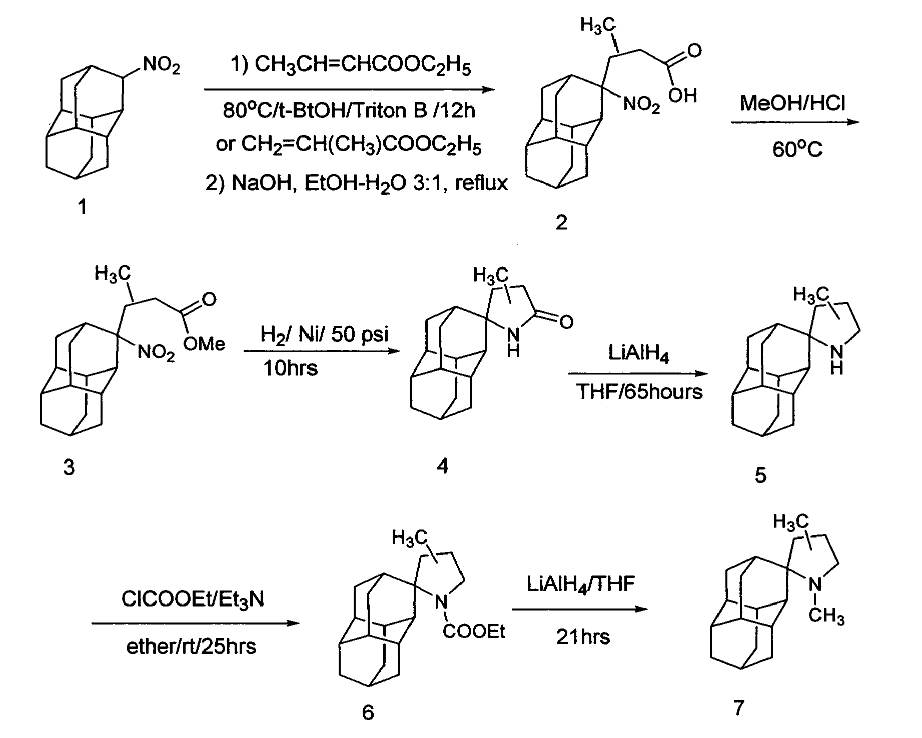

The Michael addition between 3-nitrodiamantane **1** and ethyl crotonate or methacrylate affords the corresponding ester adducts. The 3-diamantane nitroacid is converted into methyl ester. Catalytic hydrogenation over Ni-Raney gives the compound **4**, 4-methyl or 5-methylspiro[pyrrolidine-3,3'-diamantane]-5-one. Compound **4** reduces with LiA1H4 and gives the pyrrolidine-3,3'-diamantane parent compound **5**. The N-methylated (or other alkylated groups) derivatives **7** is obtained from reduction of the corresponding carbamates **6**.

### Example 3:

### General synthetic route for spiro-piperdine-diamantane derivatives

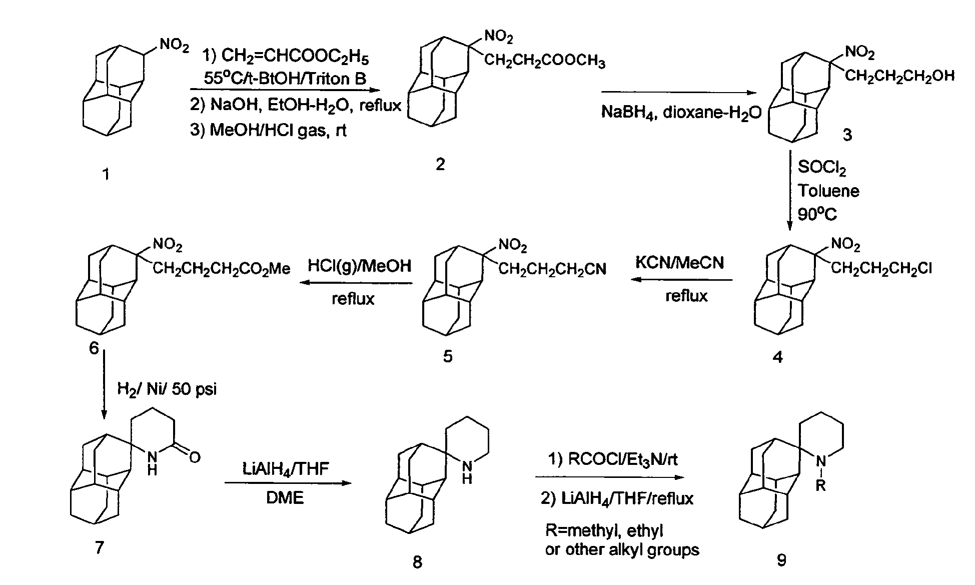

The synthetic route for spiro[piperidine-3,3'-diamantane] follows the above scheme. The Michael condensation of 3-nitrodiamantane with acrylic ethyl ester affords the corresponding ethyl ester. This is purified by saponification, and the intermediate carboxylic acid is then esterified to the methyl ester **2.** Selective reduction of the methyl ester **2** with NaBH₄ will give 3-nitro-3-diamantane propanol, compound **3.** Compound **3** reacts with SOCl₂ to give its chloride **4.** The nitrile **5** is obtained from the reaction of compound **3** with KCN in acetonitrile in the presence of 18-crown-6. Methanolysis of the nitrile **5** and hydrogenation of the intermediate nitro ester **6** over Ni-Raney catalyst affords the spiro[piperidine-3,3'diamantane]-6-one. Reduction of the compound 7 with LiA1H₄ in DME leads to the spiro[piperidine-3,3'-diamantane] **8.** N-Acrylation of the latter is followed by reduction of the intermediate amides to give the N-methyl and N-ethyl derivatives **9.**

While the present invention has been described with reference to specific embodiments, this application is intended to cover those various changes and substitutions that may be made by those of ordinary skill in the art without departing from the spirit and scope of the appended claims.

## Claims

1. A compound of Formula I: 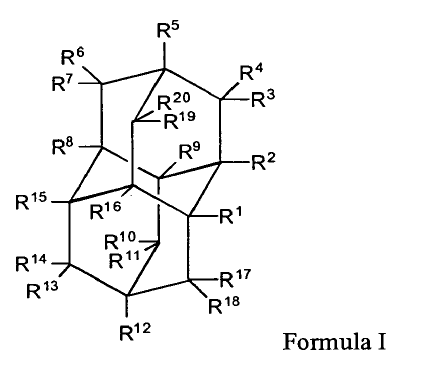 wherein:
R¹, R², R⁵, R⁸, R⁹, R¹², R¹⁵, and R¹⁶ are independently selected from the group consisting of hydrogen, hydroxy, lower alkyl, substituted lower alkyl, lower alkenyl, substituted lower alkenyl, alkoxy, amino, nitroso, nitro, halo, cycloalkyl, carboxy, acyloxy, acyl, aminoacyl, and aminocarbonyloxy;
R³, R⁴, R⁶, R⁷, R¹⁰, R¹¹, R¹³, R¹⁴, R¹⁷, R¹⁸, R¹⁹ and R²⁰ are hydrogen or R³ and R⁴, or R⁶ and k⁷, or R¹⁰ and R¹¹, or R¹³ and R¹⁴, or R¹⁷ and R¹⁸, or R¹⁹ and R²⁰, together with the carbon to which they are bonded form a 3- to 7-membered ring structure having 0, 1, 2 or 3 heteroatoms of N, O, P or S, the ring structure optionally substituted with one or more substituents independently selected from the group consisting of hydroxy, lower alkyl, substituted lower alkyl, lower alkenyl, substituted lower alkenyl, alkoxy, amino, nitroso, nitro, halo, cycloalkyl, carboxy, oxo, acyloxy, acyl, aminoacyl, and aminocarbonyloxy;
provided that at least one of R³ and R⁴, or R⁶ and R⁷, or R¹⁰ and R¹¹, or R¹³ and R¹⁴, or R¹⁷ and R¹⁸, or R¹⁹ and R²⁰, together with the carbon to which they are bonded form an optionally-substituted 3- to 7-membered ring structure;
and pharmaceutically acceptable salts thereof.

2. The compound of claim 1, wherein only one of R³ and R⁴, or R⁶ and R⁷, or R¹⁰ and R¹¹, or R¹³ and R¹⁴, or R¹⁷ and R¹⁸, or R¹⁹ and R²⁰ together with the carbon to which they are bonded forms a ring structure.

3. The compound of claim 1, wherein two or more of R³ and R⁴, or R⁶ and R⁷, or R¹⁰ and R¹¹ , or R¹³ and R¹⁴, or R¹⁷ and R¹⁸, or R¹⁹ and R²⁰, together with the carbon to which they are bonded form a ring structure, and the ring structures contain at least one heteroatom.

4. A compound of Formula II: 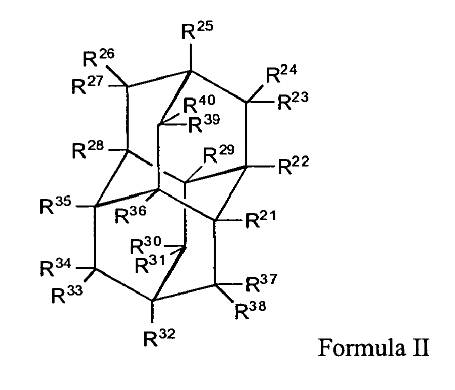 wherein:
R²¹, R²², R²⁵, R²⁸, R²⁹, R³², R³⁵, and R³⁶ are independently selected from the group consisting of hydrogen, hydroxy, lower alkyl, substituted lower alkyl, lower alkenyl, substituted lower alkenyl, alkoxy, amino, nitroso, nitro, halo, cycloalkyl, carboxy, acyloxy, acyl, aminoacyl, aminocarbonyloxy and a 3- to 7-membered ring structure having 0, 1, 2 or 3 heteroatoms of N, O, P or S, the ring structure optionally substituted with one or more substituents independently selected from the group consisting of hydroxy, lower alkyl, substituted lower alkyl, lower alkenyl, substituted lower alkenyl, alkoxy, amino, nitroso, nitro, halo, cycloalkyl, carboxy, oxo, acyloxy, acyl, aminoacyl, and aminocarbonyloxy;
provided that at least one of R²¹, R²², R²⁵, R²⁸, R²⁹, R³², R³⁵, and R³⁶ is an optionally-substituted 3- to 7-membered ring structure;
R²³, R²⁴, R²⁶, R²⁷, R³⁰, R³¹, R³³, R³⁴, R³⁷, R³⁸, R³⁹, and R⁴⁰ are hydrogen;
and pharmaceutically acceptable salts thereof.

5. The compound of claim 4, wherein a tertiary carbon of the diamantane structure is joined to R²¹, R²², R²⁵, R²⁸, R²⁹, R³², R³⁵, or R³⁶ by a carbon-carbon bond.

6. The compound of claim 4, wherein a tertiary carbon of the diamantane structure is joined to R²¹, R²², R²⁵, R²⁸, R²⁹, R³², R³⁵, or R³⁶ by a carbon-nitrogen bond.

7. The compound of claim 4, wherein only one of R²¹, R²², R²⁵, R²⁸, R²⁹, R³², R³⁵, and R³⁶ is a ring structure.

8. The compound of claim 4, wherein two or more of R²¹, R²², R²⁵, R²⁸, R²⁹, R³², R³⁵, and R³⁶ have a ring structure, and the ring structures contain at least one heteroatom.

9. A compound of Formula III: 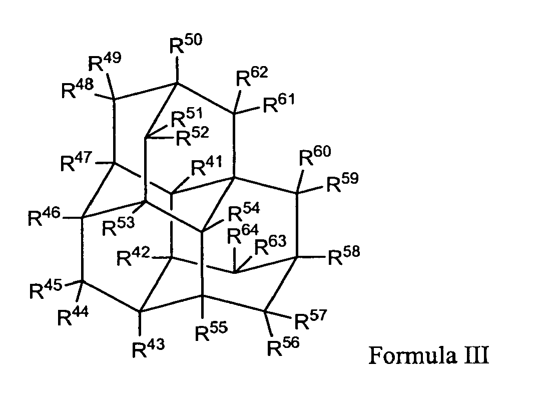 wherein:
R⁴¹, R⁴², R⁴³, R⁴⁶, R⁴⁷, R⁵⁰, R⁵³, R⁵⁴, R⁵⁵, and R⁵⁸ are independently selected from the group consisting of hydrogen, hydroxy, lower alkyl, substituted lower alkyl, lower alkenyl, substituted lower alkenyl, alkoxy, amino, nitroso, nitro, halo, cycloalkyl, carboxy, acyloxy, acyl, aminoacyl, and aminocarbonyloxy;
R⁴⁴, R⁴⁵, R⁴⁸, R⁴⁹, R⁵¹, R⁵², R⁵⁶, R⁵⁷, R⁵⁹, R⁶⁰, R⁶¹, R⁶², R⁶³, and R⁶⁴ are hydrogen or R⁴⁴ and R⁴⁵ or R⁴⁸ and R⁴⁹ or R⁵¹ and R⁵² or R⁵⁶ and R⁵⁷ or R⁵⁹ and R⁶⁰, or R⁶¹ and R⁶², or R⁶³ and R⁶⁴, together with the carbon to which they are bonded form a 3- to 7-membered ring structure having 0, 1, 2 or 3 heteroatoms of N, O, P or S, the ring structure optionally substituted with one or more substituents independently selected from the group consisting of hydroxy, lower alkyl, substituted lower alkyl, lower alkenyl, substituted lower alkenyl, alkoxy, amino, nitroso, nitro, halo, cycloalkyl, carboxy, oxo, acyloxy, acyl, aminoacyl, and aminocarbonyloxy;
provided that at least one of R⁴⁴ and R⁴⁵, or R⁴⁸ and R⁴⁹, or R⁵¹ and R⁵², or R⁵⁶ and R⁵⁷, or R⁵⁹ and R⁶⁰, or R⁶¹ and R⁶², or R⁶³ and R⁶⁴ together with the carbon to which they are bonded form an optionally-substituted 3- to 7-membered ring structure;
and pharmaceutically acceptable salts thereof.

10. The compound of claim 9, wherein only one of R⁴⁴ and R⁴⁵, or R⁴⁸ and R⁴⁹, or R⁵¹ and R⁵², or R⁵⁶ and R⁵⁷ or R⁵⁹ and R⁶⁰, or R⁶¹ and R⁶² or R⁶³ and R⁶⁴ together with the carbon to which they are bonded forms a ring structure.

11. The compound of claim 9, wherein two or more of R⁴⁴ and R⁴⁵, or R⁴⁸ and R⁴⁹, or R⁵¹ and R⁵², or R⁵⁶ and R⁵⁷, or R⁵⁹ and R⁶⁰, or R⁶¹ and R⁶² or R⁶³ and R⁶⁴, together with the carbon to which they are bonded form a ring structure, and the ring structures contain at least one heteroatom.

12. A compound of Formula IV: 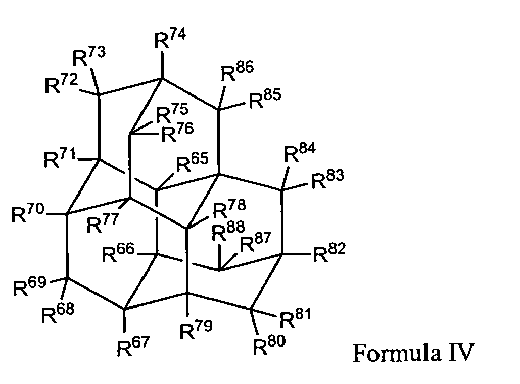 wherein:
R⁶⁵, R⁶⁶, R⁶⁷, R⁷⁰, R⁷¹, R⁷⁴, R⁷⁷, R⁷⁸, R⁷⁹, and R⁸² are independently selected from the group consisting of hydrogen, hydroxy, lower alkyl, substituted lower alkyl, lower alkenyl, substituted lower alkenyl, alkoxy, amino, nitroso, nitro, halo, cycloalkyl, carboxy, acyloxy, acyl, aminoacyl, aminocarbonyloxy and a 3- to 7-membered ring structure having 0, 1, 2 or 3 heteroatoms ofN, O, P or S, the ring structure optionally substituted with one or more substituents independently selected from the group consisting of hydroxy, lower alkyl, substituted lower alkyl, lower alkenyl, substituted lower alkenyl, alkoxy, amino, nitroso, nitro, halo, cycloalkyl, carboxy, oxo, acyloxy, acyl, aminoacyl, and aminocarbonyloxy;
provided that at least one of R⁶⁵, R⁶⁶, R⁶⁷, R⁷⁰, R⁷¹, R⁷⁴, R⁷⁷, R⁷⁸, R⁷⁹, and R⁸² is an optionally-substituted 3- to 7-membered ring structure;
R⁶⁸, R⁶⁹, R⁷², R⁷³, R⁷⁵, R⁷⁶, R⁸⁰, R⁸¹, R⁸³, R⁸⁴, R⁸⁶, R⁸⁶, R⁸⁷, and R⁸⁸ are hydrogen;
and pharmaceutically acceptable salts thereof.

13. The compound of claim 12, wherein a tertiary carbon of the triamantane structure is joined to R²¹, R²², R²⁵, R²⁸, R²⁹, R³², R³⁵, or R³⁶ by a carbon-carbon bond.

14. The compound of claim 12, wherein a tertiary carbon of the triamantane structure is joined to R²¹, R²², R²⁵, R²⁸, R²⁹, R³², R³⁵, or R³⁶ by a carbon-nitrogen bond.

15. The compound of claim 12, wherein only one of R⁶⁵, R⁶⁶, R⁶⁷, R⁷⁰, R⁷¹, R⁷⁴, R⁷⁷, R⁷⁸, R⁷⁹, and R⁸² is a ring structure.

16. The compound of claim 12, wherein two or more of R⁶⁵, R⁶⁶, R⁶⁷, R⁷⁰, R⁷¹, R⁷⁴, R⁷⁷, R⁷⁸, R⁷⁹, and R⁸² have a ring structure, and the ring structures contain at least one heteroatom.

17. The compound of claim 1, 4, 9 or 12, wherein the ring structure has 1 or 2 heteroatoms.

18. The compound of claim 17, wherein the ring structure has 1 heteroatom.

19. The compound of claim 17, wherein the ring structure has 2 different heteroatoms.

20. The compound of claim 17, wherein the ring structure has 2 heteroatoms, both heteroatoms being the same.

21. The compound of claim 1, 4, 9 or 12, wherein the ring structure is optionally substituted with lower alkyl, substituted lower alkyl, acyl, or cycloalkyl.

22. The compound of claim 21, wherein the ring structure is optionally substituted with methyl, ethyl, propyl, acetyl, -NH₂, -NH(lower alkyl) or -N(lower alkyl)₂.

23. The compound of claim 1, 4, 9 or 12, wherein the ring structure is selected from the group consisting of azirane, azirine, azetane, azete, pyrrolidine, pyrrole, piperidine, pyridine, azepane and azepine.

24. The compound of claim 1, 4, 9 or 12, wherein the ring structure is selected from the group consisting of oxirane, oxirene, oxetane, oxete, oxolane, furan, oxane, pyran, oxepane, oxepin and morpholine.

25. The compound of claim 1, 4, 9 or 12, wherein the ring structure is selected from the group consisting of thiirane, thiirene, thietane, thiete, thiole, thiophene, thiane, thiine, thiepane and thiepin.

26. The compound of claim 1, 4, 9 or 12, wherein the ring structure is selected from the group consisting of cyclopropane, cyclopropene, cyclobutane, cyclobutene, cyclopentane, cyclopentene, cyclohexane, cyclohexene, benzene, cycloheptane and cycloheptene.

27. A compound as defined in any preceding claim for treating a viral disorder in a subject in need thereof.

28. The compound for use as claimed in claim 27, wherein the viral disorder is caused by an influenza virus.

29. The compound for use as claimed in claim 28, wherein the influenza virus is an influenza A virus.

30. The compound for use as claimed in claim 29, wherein the influenza A virus has the serotype H1N1, H2N2, H3N2, H5N1, H7N7, H1N2, H9N2, H7N2, H7N3 or H10N7.

31. The compound for use as claimed in claim 30, wherein the influenza A virus has the serotype H1N1 or H3N2.

32. The compound for use as claimed in claim 28, wherein the influenza virus is an influenza B virus.

33. The compound for use as claimed in claim 27, wherein the subject is a mammal.

34. The compound for use as claimed in claim 33, wherein the mammal is a human.

35. The compound for use as claimed in claim 27, wherein the compound is administered parenterally.

36. A pharmaceutical composition for the treatment of a viral disorder comprising a pharmaceutically effective amount of the compound of any one of claims 1-26, and one or more pharmaceutically acceptable excipients or carriers.
